# EUROPEAN PATENT SPECIFICATION

(11) **EP 2 323 634 B1**
(45) Date of publication and mention of the grant of the patent: **15.11.2017**
(21) Application number: 09777576.1
(22) Date of filing: 31.07.2009
(51) Int. Cl.: A61K 9/22, A61K 9/28, A61K 31/404

(54) **ROPINIROLE COMPOSITION**
ROPINIROL-ZUSAMMENSETZUNG
COMPOSITION À BASE DE ROPINIROLE

(30) Priority: 01.08.2008 SI 200800192
(43) Date of publication of application: 25.05.2011
(73) Proprietor: KRKA, tovarna zdravil, d.d., Novo mesto, 8501 Novo mesto (SI)
(72) Inventor: ZADNIK, Jernej, 8350 Dolenjske Toplice (SI); VRECER, Franc, 8351 Straza pri Novem mestu (SI)
(74) Representative: Uexküll & Stolberg
(86) International application number: PCT/EP2009/005559
(87) International publication number: WO 2010/012482

(56) References cited:
- EP-B- 1 272 167
- WO-A-03/035042
- WO-A-2005/018605
- WO-A-2009/023761

## Description

### FIELD OF THE INVENTION

The present invention relates to novel pharmaceutical composition comprising ropinirole.

### BACKGROUND OF THE INVENTION

Ropinirole is a dopamine D₂ agonist used in the treatment of Parkinson's disease and restless legs syndrome. Ropinirole and its hydrochloride salt were first disclosed in EP113964. Later were published many more patent applications concerting ropinirole, such as EP266033, EP299602, EP300614, WO9415918, EP831810, EP1272167, EP1435921, EP1656118, WO200504011 and WO2005074387.

EP 1 272 167 describes the preparation of a multi-layer tablet, in particular a multi-layer controlled release tablet, comprising one active layer containing an active substance, hydrophilic polymeric substances, lipophilic substances and adjuvant substances; and one or more barrier layers containing hydrophilic polymeric substances, lipophilic substances, and adjuvant substances. The drawback of the multy-layered tablets according to EP 1 272 167 is, that they are rather complicated to produce, as the dissolution rate is controlled with the position of the separate layers, their thickness, as well as their composition, including many different pharmaceutical excipients.

WO 03/035042 also relates to a multi layer controlled-release tablet, comprising an active layer containing ropinirole or a pharmaceutically acceptable salt thereof, hydrophilic polymeric substances, lipophilic substances, and adjuvant substances; and one or more barrier layers containing hydrophilic polymeric substances, lipophilic substances and adjuvant substances. Again, the drawback of this invention is, that the tablets are rather complicated to produce, as the dissolution rate is controlled with the position of the separate layers, their thickness, as well as their composition, including many different pharmaceutical excipients.

WO 2005/018605 provides a controlled release oral dosage form comprising a therapeutically effective amount of ropinirole or a salt thereof, in a matrix. The first drawback of the process disclosed there is, that still it is preferable to form a double layer tablet. The second drawback of the process and compositions of WO 2005/018605 is, that the best performing formulation according to Example 8 requires a rather complicated preparation process including high-shear mixing of the active ingredient with a first part of the diluent, after which the blend is low shear-mixed with the second part of the diluent. Only after the same time consuming procedure has been applied on the second blend, can a rotary double layer press be used to form double layer tablets.

### DETAILED DESCRIPTION OF THE INVENTION

The problem solved by the present invention was thus to provide a pharmaceutical composition of ropinirole or its pharmaceutically acceptable salts which allows a straightforward, time- and money-saving process of preparing a sustained- release solid oral dosage form.

The composition according to present invention is simple for manufacturing and does not need any specially designed equipment (e.g. no rotary double layer press) as the tablets do not comprise separate layers, and enables the prolonged release of ropinirole from the composition suitable for once-a-day application to the patients.

More specifically, the present invention relates to a pharmaceutical solid matrix composition in the form of a tablet as defined in claim 1.

Further preferred embodiments of the present invention are described in the dependent claims.

Ropinirole according to present invention is preferably in salt form, especially in the form of the hydrochloride salt. More preferably ropinirole hydrochloride of crystalline form I according to WO 2005/080333 is used.

The pharmaceutical composition according to the present invention is formulated in a dosage form in the form of a tablet The composition according to the present invention is in the form of a matrix tablet which can be manufactured by direct compression or via granulation. The term matrix tablet is used to designate a tablet whose
core is a homogeneous mixture of active pharmaceutical ingredient and excipients and which does not comprise separate layers, as stated above. In other words, it means a single layer tablet.

Optional excipients which can be used in the composition additionally to above mentoned under point b and c are diluents disintegrants, hydrophobic agents, antioxidants, binders, surfactants, glidants or lubricants.

The matrix tablet core can optionally be coated with a coating improving the appearance and physical-chemical characteristics of the matrix tablet core without influence on the release profile of ropinirole.

The hydrophilic matrix former is hydroxypropylmethyl cellulose (HPMC). The hydrophilic matrix former is used in a concentration of from 40% to 60% (w/w), based on the total weight of the composition (i.e. without the coating in case of coated tablets).

Particularly preferred hydrophilic matrix formers are hydroxypropylmethyl celluloses which contain a methoxyl content of 19 to 24 % and a hydroxypropoxyl content of 4 to 12 %. These conform to the US Pharmacopeia requirement for type 2208 substitution of hypromellose. More preferred are hydroxypropylmethyl celluloses having a viscosity of 4 000 mPa.s to 250,000 mPa.s determined in 2% aqueous solution of the polymer at 20°C according to USP method such as hydroxypropymethyl celluose available under the commercial names Hypromellose K4M, Hypromellose K100M and Hypromellose K250M.

The hydrophilic release rate modifiers is a carbomer. Preferred carbomers are acrylic acid polymers usually known under their commercial name Carbopol®, especially carbomer homopolymers of type A which have a viscosity range of 4000-11000 cP (test according to USP/NF: Brookfield RVT spindle no. 5, 20 rpm, neutralized to pH 7.3 to 7.8 at 0.5wt%), such as Carbopol 71G NF and/or Carbopol 971 P. They are also characterized by a low residual content of ethyl acetate, such as below 0.5 %(w/w). Their carboxylic acid content is in the range of 52 to 68% (w/w), calculated on the dry basis.

The hydrophilic release rate modifiers is used in the concentration range 3 to 40% (w/w), preferably 10 to 30% (w/w) of the total weight of the composition.

As used herein, "release rate modifier" means substances which serve to modify the rate of release of therapeutic agents. The release rate modifier will assist in providing a controlled release of the therapeutic agent and cooperates with the hydrophilic matrix former.

Hydrophobic agents can be selected from cellulose esters such as cellulose acetate; hydrophobic cellulose ethers such as ethylcellulose; waxes and fats such as hydrogenated castor oil, hydrogenated vegetable oil, carnauba wax and microcrystalline wax; alginates such as alginic acid and sodium alginate and fatty acid derivatives such as glyceryl monostearate, glyceryl palmitostearate, glyceryl behenate or the like. Preferred hydrophobic agents are glyceryl behenate and in particular hydrogenated castor oil. Hydrophobic agents can be used in the concentration range from 5 to 40% (w/w), preferably 10 to 30% (w/w) of the total weight of the composition.

Diluents can be selected from sucrose, lactose, mannitol, dextrose, sorbitol, trehalose, starch and its derivatives, microcrystalline cellulose, calcium salts of phosphoric acid such as calcium hydrogen phosphate in anhydrous or hydrated state or a combination thereof, Diluents are preferably used in the range 5 to 50% (w/w), more preferably in the range 10 to 40% (w/w). Lactose and microcrystalline cellulose are being preferred. Also preferred are mixtures of lactose and in particular lactose monohydrate and cellulose, more preferred is a spray-dried compound consisting of alpha-lactose monohydrate and cellulose powder, most preferably a compound consisting of 70% (w/w) alpha-lactose monohydrate and 25 % (w/w) cellulose powder. Such mixtures are commercially available as Cellactose®. The most preferred diluent is lactose monohydrate.

Binders can be selected from water soluble polymers such as soluble cellulose ethers such as hydroxypropylmethyl cellulose (having a viscosity as 2% solution in water at 20°C of below 20 cps (determined according to USP method), preferably below 15 cps and most preferably below 10cps) hydroxypropyl cellulose, which contains not less than 52.0% (w/w) and not more than 81.0%(w/w) of hydroxypropoxy groups (-OCH₂CHOHCH₃), hydroxyethyl cellulose, methyl cellulose, povidone, copovidone, polyvinyl alcohol and/or water insoluble such as starch and its derivatives, microcrystalline cellulose. Binders are preferably used in an amount of up to 10% (w/w), more preferably from 1 to 8% (w/w) and most preferably from 2 to 6% (w/w).

Disintegrants can be selected from crospovidone, carboxymethylcellulose sodium, carboxymethylcellulose calcium, sodium starch glycolate, low substituted hydroxypropyl cellulose which contains not less than 5.0% (w/w) and not more than 16.0% (w/w) of hydroxypropoxy groups (-OCH₂CHOHCH₃), polacryline potassium; carboxymethylcellulose sodium being preferred.

Antioxidants can be selected from alpha tocopherol, butylated hydroxytoluene; butylated hydroxyanisole, sodium metabisulfite, potassium metabisulfite, alpha tocopherol, sodium ascorbate, ascorbic acid, ascorbyl palmitate, rutin, quercetin, coffee acid, fumaric acid, citric acid monohydrate or similar; butylated hydroxytoluene and butylated hydroxyanisole being preferred.

Surfactants can be selected from anionic surfactants such as sodium lauryl sulphate and/or non-ionic surfactants such as polysorbates, sugar esters, poloxameres or the like.

Glidants can be selected from colloidal silicon dioxide (Aerosil), talc, magnesium trisilicate, magnesium silicate, calcium phosphate, powdered cellulose or magnesium oxide.

Lubricants can be selected from metal stearates such as magnesium, calcium, zinc or aluminium stearate, sodium starch fumarate, hydrogenated vegetable oils, stearic acid.

If the total amount of ingredients is less than 100%, additional excipients are added, such as disintegrants, hydrophobic agents, antioxidants and/or glidants

Most particularly preferred compositions are:

**Composition D**

| | |
|---|---|
| ropinirole hydrochloride | 0.3 to 3.2 wt.% |
| hydroxypropylmethyl cellulose | 45-55 wt.-% |
| lactose monohydrate | 25 to 35 wt.-% |
| magnesium stearate | about 2 wt.% |
| carbomer | about 4 wt.-% |
| Hydrogenated castor oil | about 10 wt.% |
| colloidal silica | 0-1% wt.-%. |

The uncoated tablet cores preferably have a weight in the range of 300 to 600 mg and can be optionally coated with a film coating.

The drug containing core can be manufactured by the state of the art processes such as direct compression, compression of granulate obtained by the state of the art processes such as wet granulation, hot melt granulation and/or dry granulation.

In one embodiment of the present invention the ropinirole hydrochloride containing matrix tablet core is produced by direct compression of the powder mixture containing ropinirole hydrochloride, a hydrophilic matrix former and at least one further ingredient selected from hydrophilic release rate modifiers, hydrophobic agents, diluents, binders, disintegrants, antioxidants, glidants and lubricants, where hydrophilic matrix former is used in the ratio to drug from 40:1 to 190:1.

In another embodiment of the invention the ropinirole hydrochloride containing matrix tablet core can be produced via granulation, where the granulation process can be performed in the presence of solvent, such as the wet granulation processes in fluid bed, or in high shear granulators, where water and/or organic solvents, or their mixtures can be used as vehicles for the preparation of a granulation liquid, or in the absence of a granulation liquid, such as a dry granulation process, like roller compaction, or slugging, or melt granulation process. The granulation liquid for wet granulation can be composed of vehicle alone or pharmaceutical acceptable excipients selected from diluent, binder, antioxidant, surfactant can be dissolved, suspended and/or emulsified in the vehicle forming a granulation liquid.

In a special embodiment of the present invention when granulation is performed by a hot melt procedure, at least one component, preferably a binder, is used having melting or glass transition or softening point below 150°C, preferably below 120°C and most preferably in the range 35 to 90°C.

In another embodiment of the invention hot melt granulation can be performed using at least one substance, preferably a polymer with an average molecular weight of from 20,000 to 8,000,000 g/mol, preferably 30,000 to 7,000,000 g/mol, having a melting or glass transition or softening point below 200°C, preferably below 180°C. The glas transition and/or softening point of the polymer can be decreased by using a suitable plasticizer such as low molecular polyethylene glycol with an average molecular weight of below 10,000 g/mol, preferably below 8,000 g/mol, polysorbate, propylene glycol, alkyl esters of carboxylic and/or hydroxycarboxylic acids such as triethylcitrate or glycerol behenate.

The granulate is formulated by transformation of the powder mixture containing ropinirole hydrochloride, one or more hydrophilic matrix formers, one or more hydrophilic release rate modifiers, and at least one further ingredient selected from diluents, binders, disintegrants, glidants, lubricants or surfactants into granules by the before mentioned state of the art processes such as dry, wet or melt granulation. The obtained granulate is mixed with extragranular excipients selected from hydrophilic release rate agents, diluents, disintegrants, glidants and lubricants and compressed into tablet cores.

The average particle size of granulate obtained by wet granulation can be in the range of 50 to 400 µm.

Preferably, ropinirole hydrochloride and a part of the excipients are sieved and homogenously mixed in a high-shear granulator prior granulation. As the vehicle for the preparation of granulation liquid most preferably purified water is used. The water is added to the dry powder mixture by spraying nozzles, an atomizing air spraying nozzle being preferred. The resulting mixture is granulated, dried and passed through an 18-mesh sieve to give granules, which are mixed with additional excipients. The mixture is treated with a tablet machine to thereby give uncoated tablets each weighing in the range from 300-600 mg.

Low melting point binders are used in case of hot melt granulation of ropinirole. A homogenous mixture of ropinirole hydrochloride and at least one excipient selected from hydrophilic matrix formers, hydrophilic release rate modifiers, hydrophobic agents, diluents, disintegrants, antioxidants is granulated with molten or softened binder, where melting and/or softening of a binder can be achieved by in situ by heating the powder mixture including low melting binder to the melting temperature of binder or by adding of molten binder. Low melting binder can be selected from excipients having melting point below 100°C, preferably below 80°C and most preferably between 35 and 70°C such as poloxamers, polyethylene glycols with MW below 10,000 g/mol, partial glyceride, macrogol glyceride esters with fatty acids with 10 to 22 C atoms, sugar esters or the like. A polymer having a softening point of below 200°C can be selected from povidone with K values from 12 to 90, copovidone, ethylcellulose, hydroxypropylmethyl cellulose, cellulose acetate phthalate, block copolymer of polyvinyl alcohol and polyethyleneglycol, polyvinyl alcohol. An especially preferred method of granulation using softening polymers described above is melt extrusion known from the state of the art.

Hydrophilic matrix formers, hydrophilic release rate modifiers, hydrophobic agents, diluents, binders, disintegrants, antioxidants can be used exclusively intra granularly, or exclusively extra granularly or can be used partially intra and partially extra granularly.

In a special embodiment of the invention drug containing tablet core can optionally be coated with water soluble coatings based on water soluble polymers selected from cellulose ethers such as low viscosity grades of hydroxypropylmethyl cellulose, hydroxypropyl cellulose, hydroxyethyl cellulose, methyl cellulose, polyvinyl alcohol, sodium carboxymethylcellulose, block copolymer of polyvinyl alcohol and polyethyleneglycol sold as Kollicoat IR and/or Kollicoat Protect. Such coating can optionally contain further ingredients such as stabilizers, antitacking agents, plasticizers, pigments and colorants. Coating can improve physical appearance such as colour, smoothness of the surface, which is important for processability during packaging of tablets into primary packaging such as blisters of the coated tablet and easiness of swallowing of tablets by patients, it reduces sorption of water (moisture) and the diffusion of oxygen into the core. Coating can be performed by the state of the art equipment such as perforated and non-perforated coating pans and fluid bed coaters.

A preferred coating agent is based on HPMC with addition of plasticizer and opacifier which is commercially available as Opadry® coating, such as Opadry y-1-7000. The coating preferably contains pigments such as red iron oxide, yellow iron oxide and/or black iron oxide. The coating is preferably applied in an amount of 1 to 6 % (w/w), preferably 2 to 4 % (w/w) based on the weight of the tablet core. The thickness of the coating can be in the range from 5 to 50 µm, preferably 10 to 30 µm.

Particularly preferred coated compositions according to the invention are obtained by coating compositions C and D as defined above with a coating of Opadry y-1-7000 and optional addition of iron oxides.

The formulation comprising ropinirole or its salt and suitable excipients prepared according to the above described possible technological procedures exhibits, when measured by the USP Basket method (10 mesh) at 200 rpm in 500 ml of phosphate buffer pH 6.8, the following in-vitro dissolution rate:
10 - 30 % of ropinirole released by 2 hours
35 - 70 % of ropinirole released by 8 hours
55 - 85 % of ropinirole released by 12 hours and
at least 70 % of ropinirole released by 20 hours.

Humidity and water content is a highly important parameter to control in order to achieve a stable final solid dosage formulation. Consequently, the water content in the solid dosage form should be limited to be below 2.5% w/w determined by the pharmacopoeial Karl-Fisher method. Besides, the primary packaging material should therefore protect the formulation from the ambient humidity. Tablets containing ropinirole hydrochloride can be packed in air tight containers such as high density polyethylene (HDPE) containers with closure such as polypropylene (PP) closure and with or without desiccator, aluminium foil blisters or polychlorotrifluoroethylene (Aclar®) blisters or any other suitable water vapour impermeable packaging. Additionally inert gases such as nitrogen, argon or helium or vacuum can be used to assure inert atmosphere in the dosage form, such as tablet or capsule, environment in the sealed primary packaging. Inert atmosphere according to present invention mean that concentration of oxygen in the atmosphere around the solid dosage form such as tablet containing ropirinole or its salt packed in the primary packaging is less than 10 % (v/v), preferably less than 5 % (v/v) and most preferably less than 2 % (v/v). The concentration of oxygen in the atmosphere surrounding the tablet can be determined by gas chromatography.

Ropinirole used in the present invention can be prepared according to already known methods such as methods disclosed in EP113964, EP266033, EP300614, EP526529, WO9415918, WO2005040115, WO2005067922, WO2005105741 WO2005080333 EP1568689, CN1754874, WO2006123356 WO2007010557 WO2007110880, WO2007110879, CN1958570, CN1974553, WO2008075169, WO2008084499 and/or US20090043111. It can also be further recrystallized and/or purified to a purity level of more than 99%, 99.5% or 99.9%.

The invention is illustrated by reference to the following examples.

### EXAMPLES

### Example 1: - Formulation containing ropinirole hydrochloride prepared by wet granulation

Ropinirole hydrochloride, hypromellose K250M, lactose monohydrate, and hydrogenated castor oil are sieved and mixed together. To the obtained mixture purified water is added. The resulting mixture is granulated, dried and passed through an 18-mesh sieve to give granules, which were mixed with additional hypromellose K250M, Carbopol 71G NF, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 9.12 |
| Hypromellose K250M | 250.00 |
| Lactose monohydrate | 143.38 |
| Hydrogenated castor oil | 50.00 |
| Carbopol 71G NF | 37.50 |
| Magnesium stearate | 10.00 |

### Example 2: - Formulation containing ropinirole hydrochloride prepared by wet granulation

Ropinirole hydrochloride, hypromellose K250M, and lactose monohydrate, are sieved and mixed together. To the obtained mixture purified water is added. The resulting mixture is granulated, dried and passed through an 18-mesh sieve to give granules, which were mixed with additional hypromellose K250M, Carbopol 71G NF, hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 9.12 |
| Hypromellose K250M | 250.00 |
| Lactose monohydrate | 160.88 |
| Hydrogenated castor oil | 50.00 |
| Carbopol 71G NF | 20.00 |
| Magnesium stearate | 10.00 |

### Example 3: - Formulation containing ropinirole hydrochloride prepared by wet granulation

Ropinirole hydrochloride, hypromellose K100M, and lactose monohydrate, are sieved and mixed together. To the obtained mixture purified water is added. The resulting mixture is granulated, dried and passed through an 18-mesh sieve to give granules, which were mixed with additional hypromellose K100M, Carbopol 71G NF, hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 2.28 |
| Hypromellose K100M | 250.00 |
| Lactose monohydrate | 167.72 |
| Hydrogenated castor oil | 50.00 |
| Carbopol 71G NF | 20.00 |
| Magnesium stearate | 10.00 |

### Example 4: - Formulation containing ropinirole hydrochloride prepared by wet granulation

Ropinirole hydrochloride, hypromellose K100M and lactose monohydrate are sieved and mixed together. To the obtained mixture purified water is added. The resulting mixture is granulated, dried and passed through an 18-mesh sieve to give granules, which were mixed with additional hypromellose K100M, Carbopol 71G NF, polyethylene glycol (PEG 6000), colloidal silicon dioxide (Aerosil 200), hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 2.28 |
| Hypromellose K100M | 250.00 |
| Lactose monohydrate | 140.72 |
| Carbopol 71G NF | 20.00 |
| Polyethylene glycol PEG 6000 | 22.00 |
| Colloidal silicon dioxide | 5.00 |
| Hydrogenated castor oil | 50.00 |
| Magnesium stearate | 10.00 |

### Example 5: Formulation containing ropinirole hydrochloride prepared by wet granulation

Ropinirole hydrochloride, hypromellose K100M, lactose monohydrate, and colloidal silicon dioxide (Aerosil 200) are sieved and mixed together. To the obtained mixture purified water is added. The resulting mixture is granulated, dried and passed through an 18-mesh sieve to give granules, which were mixed with additional hypromellose K100M, additional colloidal silicon dioxide (Aerosil 200), Carbopol 71G NF, hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 2.28 |
| Hypromellose K100M | 250.00 |
| Lactose monohydrate | 164.72 |
| Colloidal silicon dioxide | 3.00 |
| Hydrogenated castor oil | 50.00 |
| Carbopol 71G NF | 20.00 |
| Magnesium stearate | 10.00 |

### Example 6: Coating

447.76 g of Opadry y-1-7000, 1.34 g of red iron oxide and 0.90 g of yellow iron oxide are dissolved and/or dispersed in 2550.0 g of purified water. The uncoated tablets obtained in Examples 3 and 5 are coated with above dispersion in coating pan. Thus coated tablets weighing 515.0 mg were obtained.

### Example 7: - Formulation containing ropinirole hydrochloride prepared by wet granulation

Ropinirole hydrochloride, hypromellose K100M, lactose monohydrate, colloidal silicon dioxide (Aerosil 200), and butylated hydroxytoluene are sieved and mixed together. To the obtained mixture purified water is added. The resulting mixture is granulated, dried and passed through an 18-mesh sieve to give granules, which were mixed with additional hypromellose K100M, additional colloidal silicon dioxide (Aerosil 200), Carbopol 71G NF, hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 2.28 |
| Hypromellose K100M | 250.00 |
| Lactose monohydrate | 164.67 |
| Colloidal silicon dioxide | 3.00 |
| Butylated hydroxytoluene | 0.05 |
| Hydrogenated castor oil | 50.00 |
| Carbopol 71G NF | 20.00 |
| Magnesium stearate | 10.00 |

### Example 8: Formulation containing ropinirole hydrochloride prepared by wet granulation

Ropinirole hydrochloride, hypromellose K100M, lactose monohydrate, and colloidal silicon dioxide (Aerosil 200) are sieved and mixed together. To the obtained mixture an aqueous mixture 50 % ethanol with dissolved butylated hydroxyanisole is added. The resulting mixture is granulated, dried and passed through an 18-mesh sieve to give granules, which were mixed with additional hypromellose K100M, additional colloidal silicon dioxide (Aerosil 200), Carbopol 71G NF, hydrogenated castor oil and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 2.28 |
| Hypromellose K100M | 250.00 |
| Lactose monohydrate | 164.67 |
| Colloidal silicon dioxide | 3.00 |
| Butylated hydroxyanisole | 0.05 |
| Hydrogenated castor oil | 50.00 |
| Carbopol 71G NF | 20.00 |
| Magnesium stearate | 10.00 |

### Example 9: Formulation containing ropinirole hydrochloride prepared by wet granulation

Ropinirole hydrochloride, hypromellose K100M, lactose monohydrate, hydroxypropyl cellulose (HPC Klucel EF), colloidal silicon dioxide (Aerosil 200), are sieved and mixed together. To the obtained mixture purified water is added. The resulting mixture is granulated, dried and passed through an 18-mesh sieve to give granules, which were mixed with additional hypromellose K100M, additional colloidal silicon dioxide (Aerosil 200), Carbopol 71G NF, hydrogenated castor oil, and magnesium stearate. The mixture is treated with a tablet machine to thereby give uncoated tablets of the following composition each weighing 500.0 mg.

**Composition**

| Ingredient | mg/tablet core |
|---|---|
| Ropinirole hydrochloride | 9.12 |
| Hypromellose K250M | 250.00 |
| Lactose monohydrate | 143.38 |
| Hydrogenated castor oil | 50.00 |
| Carbopol 71G NF | 37.50 |
| Magnesium stearate | 10.00 |

**Example 10:**

**Composition**

| | mg/tablet core | | | |
|---|---|---|---|---|
| Ingredient | 2 mg | 3 mg | 4 mg | 8 mg |
| Ropinirole hydrochloride | 2.28 | 3.42 | 4.56 | 9.12 |
| Hypromellose K100M | 250.00 | 250.00 | 250.00 | 250.00 |
| Lactose monohydrate | 164.72 | 163.58 | 162.44 | 157.88 |
| Hydrogenated castor oil | 50.00 | 50.00 | 50.00 | 50.00 |
| Carbopol 71 G NF | 20.00 | 20.00 | 20.00 | 20.00 |
| Aerosil 200 | 3.00 | 3.00 | 3.00 | 3.00 |
| Magnesium stearate | 10.00 | 10.00 | 10.00 | 10.00 |
| Opadry y-1-7000 | 14.925 | 13.975 | 13.585 | 13.585 |
| Red Iron Oxide | 0.045 | 0.950 | 0.283 | 0.991 |
| Yellow Iron Oxide | 0.030 | 0.045 | 0.991 | 0.283 |
| Black Iron Oxide | 0 | 0.030 | 0.141 | 0.141 |

Ropinirole hydrochloride, hypromellose K100M, lactose monohydrate, and colloidal silicon dioxide (Aerosil 200) are sieved and mixed together. To the obtained mixture purified water is added using atomizing air nozzle. The resulting mixture is granulated, dried and passed through an 18-mesh sieve to give granules, which were mixed with additional hypromellose K100M, additional colloidal silicon dioxide (Aerosil 200), Carbopol 71G NF, hydrogenated castor oil, and magnesium stearate. The mixture is treated by a tablet machine with oval 15mm x 8mm punches to thereby give uncoated tablets of the following composition each weighing 500.0 mg.

### Coating

Opadry y-1-7000, red iron oxide, yellow iron oxide and optionally black iron oxide are dissolved and/or dispersed in purified water. The uncoated tablets obtained are coated with prepared dispersion in coating pan. Thus coated tablets weighing 515.0 mg are obtained.

Ropinirole hydrochloride used in examples 1-10 was in a crystalline form I according to WO2005080333. Particle size distribution of different batches is shown in table below:

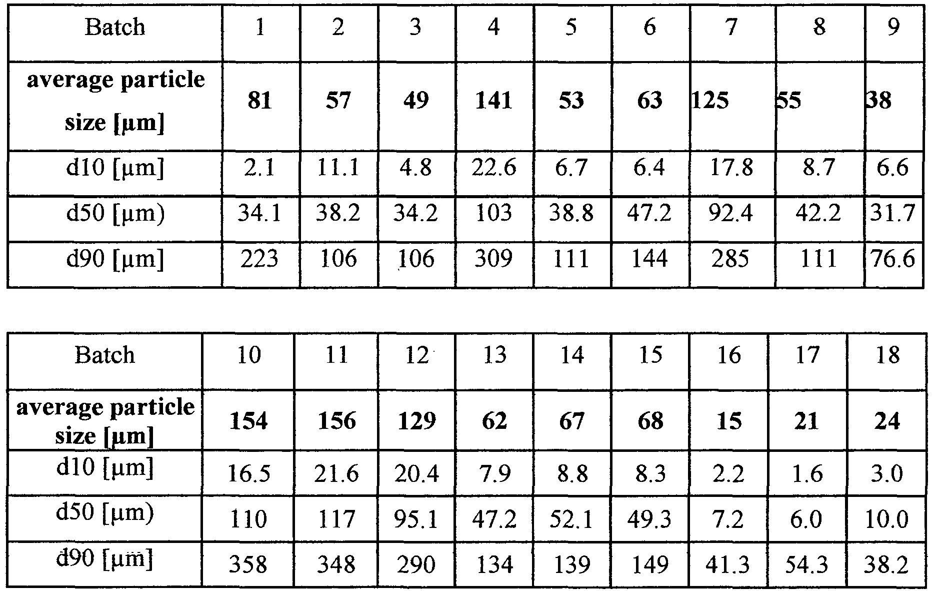

The size distribution of ropinirole hydrochloride particles was determined by laser diffraction using a Malvern Mastersizer 2000 laser diffraction instrument. The samples for analysis were prepared by dispersing a weighed amount of ropinirole hydrochloride particles in vegetable oil. With batch 1 two minute sonnication was also used. The term "average particle size" represents the mean particle diameter.

**Trade name definitions**

| **Trade name** | **Generic description Supplier** | |
|---|---|---|
| Carbopol 71G NF | Carbomer Homopolymer Type A, Carboxypolymethylene, granules | Lubrizol |
| Carbopol 971 P | Carbomer Homopolymer Type A, Carboxypolymethylene, powder | Lubrizol |
| Aerosil 200 | fumed silicon dioxide with BET surface area of 200 m2/g. | Evonik |
| PEG 6000 | polyethylene glycol with an Clariant average molecular weight of 6000 | |
| Opadry y-1-7000 | Hydroxypropylmethylcellulose, Titanium dioxide, Macrogol 400 | Colorcon |
| Hypromellose K250M | hydroxypropyl methylcellulose with molecular weight of 250000 | Dow Chemical |
| Hypromellose K100M | hydroxypropyl methylcellulose with molecular weight of 100000 | Dow Chemical |
| Cellactose | spray-dried compound consisting of 75 % alpha-lactose monohydrate and 25 % cellulose | Meggle |
| Polyox resin PEO WSR 303 | polyethylene oxide with a viscosity 7,500-10,000 mPas (1% aqueous solution at 25°C) | Dow Chemical |

## Claims

1. A pharmaceutical solid matrix composition which is present in the form of a tablet which does not comprise separate layers, said composition comprising
a) ropinirole or its salt;
b) one or more hydrophilic matrix formers, wherein the hydrophilic matrix former is hydroxypropylmethyl cellulose, wherein the concentration of the hydroxypropylmethyl cellulose matrix former is from 40 to 60% (w/w), based on the total weight of the composition without the coating in case of a coated tablet;
c) one or more hydrophilic release rate modifiers, wherein the hydrophilic release rate modifier is carbomer, wherein the concentration of the carbomer release rate modifier is from 3 to 40% (w/w), based on the total weight of the composition without the coating in case of a coated tablet; and
d) optionally other excipients,
wherein the composition has, when measured by the USP Basket method (10 mesh) at 200 rpm in 500 ml of phosphate buffer pH 6.8, the following in-vitro dissolution rate;
10 - 30 % of ropinirole released by 2 hours;
35 - 70 % of ropinirole released by 8 hours;
55 - 85 % of ropinirole released by 12 hours; and
at least 70 % of ropinirole released by 20 hours.

2. The pharmaceutical composition according to claim 1, wherein ropinirole is in the form of ropinirole hydrochloride.

3. The pharmaceutical composition according to claim 1 or claim 2, wherein hydrophilic release rate modifier is carbomer homopolymer type A.

4. The pharmaceutical composition according to any one of claims 1 to 3 comprising:
| | |
|---|---|
| ropinirole hydrochloride | 0.3 to 3.2 wt.-% |
| hydroxypropylmethyl cellulose | 45-55 wt.-% |
| lactose monohydrate | 25 to 35 wt.-% |
| magnesium stearate | 2 wt.-% |
| carbomer | 4 wt.-% |
| hydrogenated castor oil | 10 wt.-% |
| colloidal silica | 0-1 wt.-%. |

5. The composition of any one of the previous claims comprising a water soluble coating.

6. The composition of claim 5, wherein the coating is selected from cellulose ethers, low viscosity grades of hydroxypropylmethyl cellulose, hydroxypropyl cellulose, hydroxyethyl cellulose, methyl cellulose polyvinyl alcohol, sodium carboxymethyl cellulose.

## Patentansprüche

1. Pharmazeutische Feststoffmatrixzusammensetzung, die in Form einer Tablette vorliegt, die keine getrennten Schichten umfasst, wobei die Zusammensetzung
a) Ropinirol oder dessen Salz,
b) einen oder mehrere hydrophile Matrixbildner, wobei der hydrophile Matrixbildner Hydroxypropylmethylcellulose ist, wobei die Konzentration des Hydroxypropylmethylcellulosematrixbildners von 40 bis 60% beträgt (Gew./Gew.), bezogen auf das gesamte Gewicht der Zusammensetzung ohne die Beschichtung im Falle einer beschichteten Tablette,
c) eines oder mehrere hydrophile Mittel zur Modifizierung der Freisetzungsrate, wobei das hydrophile Mittel zur Modifizierung der Freisetzungsrate Carbomer ist, wobei die Konzentration des Carbomermittels zur Modifizierung der Freisetzungsrate von 3 bis 40% beträgt (Gew./Gew.), bezogen auf das Gesamtgewicht der Zusammensetzung ohne die Beschichtung im Falle einer beschichteten Tablette, und
gegebenenfalls andere Hilfsmittel umfasst,
wobei die Zusammensetzung die folgende *in-vitro*-Auflösungsrate aufweist, wenn mit der USP-Korb-Methode (10 Mesh) bei 200 UpM in 500ml Phosphatpuffer bei pH 6,8 gemessen:
nach zwei Stunden 10 bis 30% freigesetztes Ropinirol,
nach acht Stunden 35 bis 70% freigesetztes Ropinirol,
nach 12 Stunden 55 bis 85% freigesetztes Ropinirol und
nach 20 Stunden mindestens 70% freigesetztes Ropinirol.

2. Pharmazeutische Zusammensetzung nach Anspruch 2, bei der Ropinirol in Form von Ropinirolhydrochlorid vorliegt.

3. Pharmazeutische Zusammensetzung nach Anspruch 1 oder Anspruch 2, bei der das Mittel zur Modifizierung der Freisetzungsrate Carbomerhomopolymer Typ A ist.

4. Pharmazeutische Zusammensetzung nach einem der Ansprüche 1 bis 3, die:
0,3 bis 3,2 Gew.-% Ropinirolhydrochlorid,
45 bis 55 Gew.-% Hydroxypropylmethylcellulosefilm,
25 bis 35 Gew.-% Lactosemonohydrat,
2 Gew.-% Magnesiumstearat,
4 Gew.-% Carbomer,
10 Gew.-% hydriertes Rhizinusöl,
0 bis 1 Gew.-% kolloidales Siliciumdioxid umfasst.

5. Zusammensetzung nach einem der vorhergehenden Ansprüche, die eine wasserlösliche Beschichtung umfasst.

6. Zusammensetzung nach Anspruch 5, bei der die Beschichtung ausgewählt ist aus Celluloseethern, Hydroxypropylmethylcellulose mit niedrigen Viskositäten, Hydropxypropylcellulose, Hydroxyethylcellulose, Methylcellulosepolyvinylalkohol, Natriumcarboxymethylcellulose.

## Revendications

1. Composition de matrice solide pharmaceutique qui se présente sous la forme d'un comprimé qui ne comprend pas de couches séparées, ladite composition comprenant :
a) du ropinirole ou son sel ;
b) un ou plusieurs agents de formation de matrice hydrophiles, où l'agent de formation de matrice hydrophile est l'hydroxypropylméthylcellulose, où la concentration de l'agent de formation de matrice hydroxypropylméthylcellulose est comprise entre 40 et 60 % (p/p), par rapport au poids total de la composition sans revêtement dans le cas d'un comprimé revêtu ;
c) un ou plusieurs modificateurs de vitesse de libération hydrophiles, où le modificateur de vitesse de libération hydrophile est un carbomère, où la concentration du modificateur de vitesse de libération carbomère est comprise entre 3 et 40 % (p/p), par rapport au poids total de la composition sans revêtement dans le cas d'un comprimé revêtu ; et
d) éventuellement d'autres excipients,
la composition, lors de mesure par le procédé dit « USP Basket » (10 mesh) à 200 tr/min dans 500 ml de tampon phosphate à pH 6,8, présentant la vitesse de dissolution in vitro suivante :
10 à 30 % de ropinirole libérés par 2 heures ;
35 à 70 % de ropinirole libérés par 8 heures ;
55 à 85 % de ropinirole libérés par 12 heures ; et
au moins 70 % de ropinirole libérés par 20 heures.

2. Composition pharmaceutique selon la revendication 1, dans laquelle le ropinirole se présente sous forme de chlorhydrate de ropinirole.

3. Composition pharmaceutique selon la revendication 1 ou 2, dans laquelle le modificateur de vitesse de libération hydrophile est un homopolymère carbomère de type A.

4. Composition pharmaceutique selon l'une quelconque des revendications 1 à 3, comprenant :
| | |
|---|---|
| du chlorhydrate de ropinirole | 0,3 à 3,2 % en poids |
| de l'hydroxypropylméthylcellulose | 45 à 55 % en poids |
| du lactose monohydraté | 25 à 35 % en poids |
| du stéarate de magnésium | 2 % en poids |
| un carbomère | 4 % en poids |
| de l'huile de ricin hydrogénée | 10 % en poids |
| de la silice colloïdale | 0 à 1 % en poids. |

5. Composition de l'une quelconque des revendications précédentes, comprenant un revêtement hydrosoluble.

6. Composition de la revendication 5, dans laquelle le revêtement est choisi parmi des éthers de cellulose, des types à basse viscosité d'hydroxypropylméthylcellulose, l'hydroxypropylcellulose, l'hydroxyéthylcellulose, la méthylcellulose, l'alcool polyvinylique, la carboxyméthylcellulose sodique.
